# FASCICULE DE BREVET EUROPEEN

(11) **EP 1 178 847 B1**
(45) Date de publication et mention de la délivrance du brevet: **17.01.2007**
(21) Numéro de dépôt: 00922410.6
(22) Date de dépôt: 17.05.2000
(51) Int. Cl.: A61L 27/12, A61F 2/28

(54) **SUBSTITUT OSSEUX DE TYPE PHOSPHATE DE CALCIUM**
KNOCHENERSATZ AUS KALZIUMPHOSPHAT
CALCIUM PHOSPHATE BONE SUBSTITUTE

(30) Priorité: 19.05.1999 CH 93599
(43) Date de publication de la demande: 13.02.2002
(73) Titulaire: ECOLE POLYTECHNIQUE FEDERALE DE LAUSANNE (EPFL), 1015 Lausanne (CH)
(72) Inventeur: LEMAITRE, Jacques, CH-1015 Lausanne (CH); TERRAZZONI, Stéphane, CH-1015 Lausanne (CH)
(74) Mandataire: Besse, François
(86) Numéro de dépôt international: PCT/CH2000/000275
(87) Numéro de publication internationale: WO 2000/071178

(56) Documents cités:
- EP-A- 0 551 611
- WO-A-97/17285
- US-A- 5 525 148
- US-A- 5 531 794

## Description

### Domaine de l'invention

La présente invention concerne un substitut osseux de forme anatomique et de macroporosité contrôlées réalisé à partir de matériaux phosphocalciques cimentaires consolidés chimiquement. Le substitut peut être obtenu par mise en forme d'une pâte liquide ou plastique constituée de phosphate de calcium et d'autres sels de calcium peu solubles. La mise en forme par moulage utilisée avec une pâte et sa consolidation chimique ex-vivo permet d'obtenir d'une part ; un substitut de taille plus ou moins importante dont la forme anatomique contrôlable correspond à celle du bloc osseux à remplacer; d'autre part une architecture macroporeuse adaptée qui favorise la réhabitation et la résorption du substitut, tout en garantissant les meilleures propriétés mécaniques initiales et leur maintien jusqu'au remplacement définitif de la greffe par l'os néoformé. L'invention concerne également différents procédés permettant la réalisation de moule et de la phase porogène qui définissent respectivement la géométrie et l'architecture macroporeuse du substitut lors de la mise en forme par coulage ou par injection d'une pâte phosphocalcique.

Ce type de substitut peut être utilisé comme greffe artificielle osseuse (objet résorbable) ou comme implant osseux (objet non-résorbable), et ceci dans un large domaine d'applications en raison de sa composition et de ses caractéristiques macroscopiques (taille, forme anatomique) et microscopiques (architecture poreuse, microporosité) parfaitement adaptables. Il peut également servir de support pour la culture de cellules osseuses in vitro.

### Etat de la technique

Dans le cadre de réparation et de remplacement des tissus durs du squelette, les chirurgiens en orthopédie et traumatologie sont de plus en plus incités à rechercher de nouveaux matériaux synthétiques capables de remplacer avantageusement les greffes osseuses classiques. En effet, le champ d'application des autogreffes comme substituts osseux se limite à des comblements de faible taille ; les allogreffes peuvent entraîner des problèmes de tolérance immunologique (transmission de maladie virale) et de législation ; les xénogreffes osseuses s'accompagnent parfois de risques de transmissions infectieuses. Dès lors, des études ont été menées sur des matériaux synthétiques de type phosphate de calcium dont la composition chimique est très proche de la phase minérale de l'os ce qui les rend parfaitement biocompatibles. De plus, contrairement aux biomatériaux inertes comme l'alumine, ils sont bioactifs et ostéoconducteurs c'est à dire qu'ils favorisent les échanges entre les cellules et le tissus osseux receveur ainsi que l'ostéogenèse. Ces matériaux phosphocalciques se présentent principalement sous forme de céramiques ou de ciments.

Il existe trois principales compositions de biocéramiques (de Groot et coll. 1983). Les céramiques HAp sont peu résorbables en site biologique (Jarcho et coll. 1981). Les céramiques β-TCP sont plus solubles et leur résorption au contact de fluides biologiques a été démontrée (Eggli et coll. 1988). Enfin, il existe les biocéramiques biphasées, mélanges de HAp et de β-TCP, dont les propriétés intermédiaires varient en fonction des proportions des deux constituants du mélange (LeGeros et coll. 1988). Le comportement biologique des biocéramiques, en particulier la biodégradation, dépend des caractéristiques physico-chimiques du biomatériau (essentiellement du rapport Ca/P). Il a été également montré que l'introduction de macropores accélère les processus de recolonisation du substitut par l'os adjacent ainsi que la repousse de l'os néoformé. En outre, il semblerait que la dimension des macropores ainsi que la taille et le nombre d'interconnexions entre les macropores influencent fortement les processus de réhabitation osseuse (Fabbri et coll. 1994). Ainsi, de nombreux procédés ont été étudiés pour synthétiser des biocéramiques à architecture macroporeuse plus ou moins contrôlée. Généralement, les céramiques macroporeuses sont réalisées par addition d'agents porogènes (particules de naphtalène, camphre, polyéthylène, PVB...) lors de la mise en forme par coulage en barbotine ou par pressage à sec (Jarco et coll. 1981; Driskell et coll. 1973). Ces particules porogènes se décomposent avant la densification laissant leurs empreintes sous forme de pores dans la céramique.

De son coté, White et Schors (1986) ont développé un procédé permettant d'utiliser le corail comme porogène. D'autres techniques, telles que celles décrites dans la demande de brevet européen EP-A-253506 ou la demande WO 98/38949, sont également envisageables. Il est également possible de provoquer l'échange entre les ions CO₃²⁻ du corail et les ions PO₄³⁻ d'une solution de phosphate sous haute pression et haute température. La céramique obtenue a la structure cristallographique de l'HAp et l'architecture poreuse du corail. Cependant, il est à noter que les céramiques phosphocalciques demeurent biologiquement peu actives en raison de leur mode de consolidation par frittage à haute température. Il est donc indispensable d'introduire une forte fraction volumique poreuse pour accélérer de manière significative les processus de réhabitation et surtout de biorésorption. De plus, les procédés de mise en forme classiques ne permettent pas de réaliser des céramiques dont l'architecture macroporeuse soit contrôlable à volonté. En effet, il s'avère souvent difficile de définir indépendamment la fraction volumique et la géométrie de la macroporosité. Ceci s'illustre particulièrement dans le procédé utilisant comme agents porogènes des billes de naphtalènes. Dans ce cas précis, il est nécessaire d'introduire un volume macroporeux très important pour obtenir des interconnexions entre les macropores dont le nombre et la taille permettent la réhabitation osseuse. Tout ceci a pour conséquence de diminuer fortement les propriétés mécaniques des pièces macroporeuses. Ainsi, les applications cliniques pour lesquelles ce type de greffe subirait des sollicitations mécaniques importantes sont à déconseiller.

Les matériaux phosphocalciques se présentent aussi sous forme de ciment. Les formulations les plus couramment étudiées sont : les mélanges de phosphate tétracalcique (TTCP) et de phosphate dicalcique dihydraté (DCPD) (BoneSource^{®} HA cement), les mélanges de phosphate tricalcique-α (α-TCP) de phosphate monocalcique monohydraté (MCPM) et de carbonate de calcium (Norian SRS^{®}), ceux de carbonate de calcium et de phosphate dicalcique anhydre (DCP) et enfin les mélanges à base de phosphate tricalcique-β (β-TCP) et de MCPM (Mirtchi A. A., Lemaître J., & Terao N., Biomaterials, 1989). Leur résorbabilité et leur caractère ostéoconducteur en font des matériaux prometteurs de greffe osseuse artificielle. Les ciments phosphocalciques hydrauliques sont soit employés sous forme de pâte injectable pour comblement osseux temporaire dans le cadre d'interventions chirurgicales mini-invasives ; soit utilisés comme greffe osseuse sous forme de pièces prédurcies ex-vivo. Leur mode de consolidation chimique à basse température, c'est-à-dire inférieure à 900 °C, permet d'obtenir des substituts biologiquement plus actifs que les céramiques de compositions similaires consolidées par frittage à haute température. En effet, ces matériaux conservent une microporosité intrinsèque plus importante qui leur confère une bioactivité supérieure à celles des biocéramiques. En revanche, leur faible compacité diminue leurs propriétés mécaniques.

Les brevets US-A-5714103 et EP-A-0551611 décrivent des objets qui peuvent être utilisés comme implants osseux à base de ciment phosphocalcique. Il est constitué d'une pluralité de couches qui se superposent progressivement lors de la réalisation de l'objet. Ici, la macroporosité de l'objet ressemble à l'architecture spongieuse naturelle de l'os.

Les objets à base de ciment phosphocalcique de l'état de la technique présentent cependant plusieurs inconvénients. En particulier, leur macroporosité ne peut être contrôlée de manière suffisamment précise.

### Description de l'invention

La présente invention a notamment le mérite de remédier au problème susmentionné. Elle se rapporte à un substitut osseux de forme anatomique et d'architecture poreuse contrôlées obtenus à partir de matériaux phosphocalciques cimentaires ainsi qu'à différents procédés permettant sa ré alisation.

Plus précisément, la présente invention concerne un substitut osseux selon la revendication 1.

La mise en forme peut se faire soit par coulage d'une pâte liquide, soit par injection d'une pâte plastique, dans un moule approprié. La pâte est constituée principalement d'un mélange de phosphates de calcium auquel peuvent s'ajouter d'autres sels de calcium peu solubles ainsi que divers additifs inorganiques ou organiques régulateurs de prise, déflocculants, plastifiants. Un soin particulier est apporté lors de la préparation de la pâte afin d'obtenir les propriétés de coulabilité et de mouillabilité nécessaires à un bon moulage. Le moule permettant de définir la géométrie anatomique du substitut peut être réalisé soit en caoutchouc de silicone ou en plâtre à l'aide d'un contre-moule, soit à l'aide des procédés courants de prototypage rapide. Par ailleurs, différentes techniques permettent de générer lors de la mise en forme une architecture poreuse contrôlable à volonté, calculée pour favoriser la réhabitation et la résorption du substitut, tout en garantissant les meilleures propriétés mécaniques initiales et leur maintien dans le temps. La phase porogène qui définit l'architecture macroporeuse à introduire peut être obtenue, par exemple, à l'aide de fils polymères dans les cas les plus simples ou à l'aide de procédés de prototypage rapide pour des géométries plus complexes. Un choix judicieux de la formulation de la pâte de ciment permet de maintenir des changements dimensionnels survenant lors de la consolidation chimique à un niveau négligeable, ce qui permet de fabriquer les pièces directement aux cotes exactes. De plus, ce mode de consolidation par voie chimique permet d'obtenir des pièces biologiquement actives. Ces substituts peuvent être utilisés comme greffes artificielles osseuses dans un large domaine d'application en raison de leurs compositions et de leurs caractéristiques macroscopiques (taille, forme anatomique) et microscopiques (architecture poreuse, microporosité) parfaitement adaptables.

Le substitut en ciment phosphocalcique peut être réalisé à partir d'une pâte liquide ou plastique constituée de phosphates de calcium et d'autres sels de calcium peu solubles, auxquelles peuvent s'ajouter divers additifs minéraux ou organiques (régulateurs de prise, déflocculants, plastifiants). Le principe consiste en un mélange en milieu aqueux de deux poudres coréactives, l'une acide et l'autre basique, qui provoque la dissolution des deux réactifs et la précipitation d'une nouvelle phase thermodynamiquement plus stable. Dans le cadre de cette invention, diverses formulations connues de ciments phosphocalciques peuvent être envisagées. Par exemple, les ciments de type brushite obtenus à partir de mélanges de phosphate tricalcique-β et de phosphate monocalcique monohydraté consolident rapidement à température ambiante et possèdent une résorbabilité élevée (Mirtchi A. A., Lemaître J., & Terao N., Biomaterials, 1989). Par ailleurs, un nouveau ciment hydroxyapatite, obtenu par étuvage en atmosphère saturée d'humidité de mélange de monétite (phosphate dicalcique anhydre) et de carbonate de calcium (calcite, aragonite ou vatérite), a été spécialement développé pour cette invention. Ce ciment est particulièrement adapté pour la réalisation de substituts par coulage de pâte liquide à partir de moules et de phases porogènes obtenues par des procédés de prototypage rapide (cf. exemple 2.2). D'autres formulations déjà connues (Norian SRS^{®}, BoneSource^{®} HA cement, α-BSM^{™} Etex Corporation, CEMENTEK^{®} Teknimed,...) sont également utilisables dans le cadre de cette invention.

Le substitut faisant l'objet de la présente invention possède une forme anatomique contrôlée avec une définition très précise des dimensions. La taille du substitut peut varier de quelques millimètres à plusieurs dizaines de centimètres. Sa forme est étudiée de manière à reproduire l'anatomie de l'os à remplacer ; elle reprend en particulier les mêmes cotes externes, facilitant ainsi son insertion dans le site receveur. Par exemple, dans le cas d'os longs tels que les diaphyses osseuses, un canal central préfigurant le canal médullaire peut être ménagé selon l'axe du substitut, ceci dans le but d'accélérer la vascularisation du bloc après substitution.

L'architecture poreuse du substitut peut être constituée d'un réseau principal de pores ouverts de forme allongée (canaux) auquel peut s'ajouter selon le cas, un ou deux réseaux poreux secondaires interconnectés au premier réseau. Le réseau principal de pores allongés peut être un réseau de type carré ou aléatoire, de préférence hexagonal. Il peut être orienté à volonté, par exemple parallèlement aux sollicitations mécaniques principales que doit supporter le substitut : ceci permet d'optimiser les performances mécaniques de l'objet. Les pores du réseau principal se présentent sous forme de canaux allongés et parallèles entre eux. Par exemple, ces canaux peuvent être de forme approximativement cylindrique. L'intersection de ces canaux allongés avec un plan perpendiculaire à leur direction forme des sections de surface constante dont les centres d'inertie sont distants de 0.6 à 2 mm de préférence. La section constante des canaux a un périmètre qui se présente d'une manière générale sous la forme d'une courbe fermée convexe approximativement circulaire. La surface occupée par la section constante des canaux est comprise entre 8 10⁻³ et 0.8 mm², de préférence entre 0.1 et 0.3 mm². Un ou deux réseaux de pores secondaires relient les pores allongés du réseau principal pour faciliter les échanges métaboliques entre le substitut et l'environnement physiologique. Ils se présentent sous forme de trous pouvant être de forme cylindrique, dont la section constante est comprise entre 3 10⁻³ et 8 10⁻³ mm². Les centres d'inertie des sections constantes des pores peuvent être distants de 1.1 à 2.1 mm de préférence. L'architecture macroporeuse interconnectée occupe selon le cas une fraction volumique comprise entre 10 et 70%, de préférence entre 30 et 60% du volume total apparent. De plus, s'ajoute à celle-ci la microporosité intrinsèque du ciment phosphocalcique qui occupe selon la formulation environ 20-60% du volume total. En définitive, le substitut a une porosité totale comprise entre 28 et 88% en volume.

La réalisation du substitut se fait ex-vivo. Selon un mode préférentiel de l'invention, tout d'abord, la préparation de la pâte (plastique ou liquide) contenant les poudres co-réactives fait l'objet d'un soin tout particulier afin d'obtenir des propriétés de coulabilité et de mouillabilité nécessaires à un bon moulage. Selon le cas, la rhéologie et l'homogénéité de la pâte peuvent être optimisées par addition de polymères hydrosolubles de préférence biocompatibles (acide polyacrylique, etc...). Différents traitements sont également utilisés pour broyer ou désagglomérer les poudres co-réactives afin de mieux les disperser et ainsi homogénéiser les pâtes.

La mise en forme du substitut se fait par coulage ou par injection dans un moule. Selon la forme anatomique désirée, il existe différents procédés permettant d'obtenir le moule dans lequel sera coulée ou injectée la pâte de ciment. Pour des géométries simples, les moules peuvent être réalisés directement en caoutchouc de silicone ou en plâtre à partir d'un contre-moule en acier préalablement usiné. Il est également possible de fabriquer des moules de géométries complexes à l'aide d'un procédé de prototypage. Le procédé de prototypage rapide par jet de cire de type ModelMaker II commercialisé par Sanders Prototype Inc, USA, est particulièrement approprié. Dans ce cas précis, la forme du moule est conçue à partir d'images en 3 dimensions définies par logiciel CAD ou directement à partir d'images numérisées. Le transfert de données au logiciel asservissant la machine de prototypage peut se faire sous tout format connu, par exemple, .IGES, .STL, .DXF, .HPP, .OBJ. Le logiciel utilise les informations pour piloter un jet de cire dans le plan horizontal afin de réaliser le moule par couches successives. La phase porogène destinée à générer la macroporosité finale, peut être réalisée directement en fil de polymère pour les architectures simples ou par le procédé de prototypage rapide pour les architectures macroporeuses plus complexes. Dans ce dernier cas, la phase porogène est réalisée en même temps que le moule et est intégrée à celui-ci.

Après moulage, la consolidation ex-vivo des pièces se produit par réaction chimique à des températures pouvant varier de préférence entre la température ambiante et 121°C. L'étape de démoulage peut se faire avant ou après consolidation chimique, en fonction de la formulation du ciment et la nature du porogène et des matériaux de moulage. Enfin, la phase porogène peut être éliminée soit par simple extraction mécanique (cas des fils polymères) soit par décomposition thermique à une température inférieure à 900°C, attaque chimique ou dissolution dans un solvant approprié.

Les avantages d'un substitut anatomique à architecture macroporeuse contrôlée sont multiples. Tout d'abord, en raison ses caractéristiques (composition, forme, macroporosité) parfaitement contrôlées et de se tenue mécanique optimisée, le substitut peut être utilisé comme substitut osseux synthétique dans de nombreuses applications cliniques. Il est possible de réaliser des pièces dont la géométrie et la macroporosité sont optimisées de manière à obtenir des propriétés mécaniques proches de celles de l'os à remplacer en particulier le module élastique, tout en favorisant une résorption et une recolonisation rapides par du tissus osseux nouveau. On peut donc envisager son emploi comme macrogreffe dans les cas, par exemple, d'allongement ou de remplacement d'os longs ou encore de fusion lombaire pour lesquels la greffe doit supporter rapidement d'importantes sollicitations mécaniques.

Le substitut peut également être employé après mise en milieu de culture de cellules osseuses in vitro. Cette étape permet d'amorcer la recolonisation de l'architecture macroporeuse et de la microporosité par du tissu osseux afin de préconsolider la pièce avant son implantation en milieu biologique. Le milieu de culture peut contenir diverses molécules actives, par exemple des antibiotiques ou des facteurs de croissance osseuse (Bone Morphological Proteins) dans le but de stimuler la prolifération des cellules osseuses au coeur du substitut. Il est également possible d'utiliser le substitut comme simple support de culture de cellules osseuses.

Contrairement aux ciments injectables, le matériau est prédurci ex-vivo. Dans ces conditions, il est possible de contrôler à volonté les caractéristiques du substitut c'est à dire : sa forme anatomique, en utilisant un moule de géométrie plus ou moins complexe facile à réaliser ; son architecture poreuse, en introduisant une phase porogène à architecture définie et optimisée. L'étape de consolidation chimique contrairement au frittage des céramiques, s'effectue à faible température et n'entraîne pas de variation sensible des dimensions de la pièce. La forme et l'architecture poreuse du substitut sont ainsi définies dès le moulage et ne changent pas lors de la consolidation. De plus, le mode de consolidation chimique confère au substitut un caractère hautement bioactif contrairement aux compositions similaires consolidées par frittage à haute température. Le substitut conserve ainsi une microporosité importante qui favorise la biorésorption et permet son imprégnation avec des molécules actives diverses (antibiotiques, facteurs de croissance osseuse...). Il n'est donc pas nécessaire d'introduire une importante macroporosité pour améliorer de manière significative la réhabitation osseuse du matériau ce qui permet de minimiser l'affaiblissement mécanique du substitut.

Les techniques de mise en forme développées pour ce type de substitut permettent de générer une architecture poreuse parfaitement contrôlée et optimisée sur les plans biomécanique et biologique. Par exemple, le réseau principal poreux peut être orienté parallèlement aux sollicitations mécaniques que subit le substitut une fois en place, ce qui améliore la tenue mécanique initiale de l'ensemble. Par ailleurs, le substitut peut posséder une macroporosité totalement interconnectée, favorable à une recolonisation osseuse rapide, malgré une fraction volumique poreuse faible. Ceci garantit des propriétés mécaniques élevées contrairement aux substituts céramiques macroporeux classiques pour lesquels la recherche d'une interconnexion suffisante entre pores nécessite une fraction poreuse totale importante souvent de l'ordre de 70%. Par ailleurs, la macroporosité est étudiée de manière à accélérer la réhabitation de la greffe avant que les processus de biorésorption n'interviennent. Ceci permet un renforcement mécanique rapide par l'os néoformé et un bon maintien des propriétés fonctionnelles de la greffe au cours des premières semaines qui suivent à la mise en place du substitut. Ainsi, cette macroporosité réduite à géométrie optimisée favorise une réhabitation osseuse rapide sans sacrifier de manière importante la tenue mécanique du substitut.

Par ailleurs, les différents procédés mis en oeuvre pour la réalisation de moules et de phases porogènes, soit par exemple pour un moule en caoutchouc de silicone ou en plâtre et une phase porogène en fil polymère, soit avec un prototypage rapide, permettent d'obtenir des géométries et des architectures poreuses contrôlables à volonté. Plus particulièrement, le procédé par prototypage rapide par jet de cire présente plusieurs avantages. Tout d'abord, la conception des pièces (phase porogène et moule) est assistée par ordinateur ce qui permet d'en améliorer la rapidité et la flexibilité : par exemple, la forme anatomique peut être définie à partir d'images médicales numérisées et l'architecture macroporeuse interconnectée peut être modélisée afin d'optimiser simultanément les propriétés mécaniques finales du substitut et la recolonisation osseuse. De plus, ce procédé permet de réaliser des pièces de formes très complexes avec une haute précision dimensionnelle.

L'invention est illustrée ci-après au moyen des figures suivantes :
- La figure 1: représente un substitut osseux selon un premier mode de réalisation de l'invention.
- La figure 2: montre le substitut de la figure 1 vu de dessus et une coupe de face.
- La figure 3: illustre un moule pour réaliser le substitut des figures 1 et 2.
- La figure 4: montre une partie du dispositif pour réaliser la macroporosité du substitut des figures précédentes.
- La figure 5: montre l'autre partie de ce même dispositif.
- La figure 6: illustre l'ensemble de ce même dispositif vu en perspective.
- La figure 7: montre ce même dispositif avec des fils en polymère.
- La figure 8: montre ce même ensemble, dans une position, avec le moule des figures précédentes.
- La figure 9: montre l'ensemble de la figure 8 dans une autre position avec l'implant.
- La figure 10: représente un substitut osseux selon un deuxième mode de réalisation de l'invention.
- La figure 11: montre le substitut de la figure 10 vu de dessus et une coupe de face.
- La figure 12: illustre un moule pour réaliser le substitut des figures 10 et 11.
- La figure 13: illustre ce même moule vu en perspective.
- La figure 14: montre l'ensemble constitué par le moule et le dispositif pour réaliser la macroporosité du substitut des figures 10 et 11.

### EXEMPLE 1 : SUBSTITUT CYLINDRIQUE A ARCHITECTURE

### MACROPOREUSE UNIDIRECTIONNELLE OU BIDIRECTIONNELLE REALISE A PARTIR DE CIMENT DE TYPE BRUSHITE.

Le substitut est mis en forme par injection d'une pâte liquide en ciment brushitique dans un moule en caoutchouc de silicone (macroporosité unidirectionnelle) ou en acier inoxydable (macroporosité bidirectionnelle). L'architecture macroporeuse est réalisée par moulage à partir de fils en polymère (p.ex. nylon) ou en acier inoxydable (bidirectionnelle).

### a) Substitut à architecture macroporeuse unidirectionnelle.

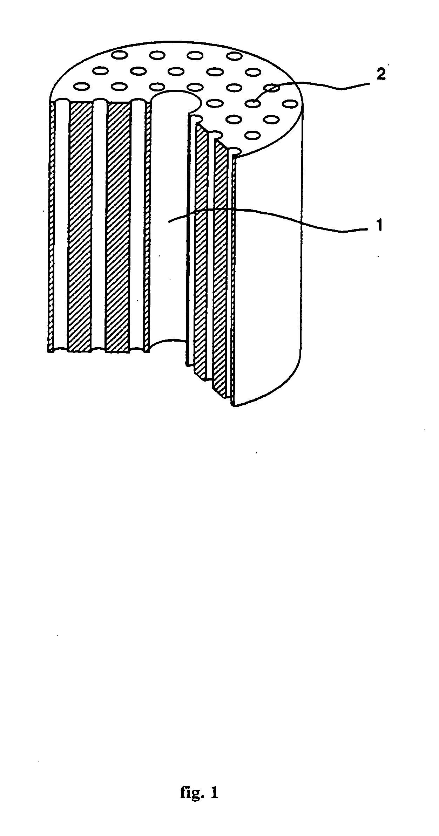

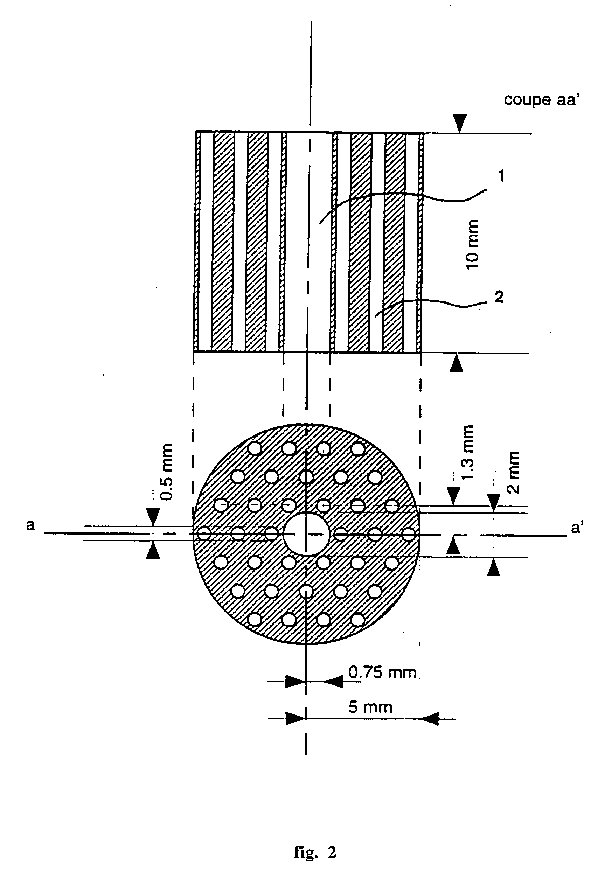

### Description du substitut (fig. 1 et 2)

Le substitut se présente sous forme d'un cylindre de 10 mm de diamètre et 10 mm de hauteur. Un canal central (1) de 2 mm de diamètre est pratiqué selon l'axe du substitut et simule la cavité médullaire de l'os à remplacer. L'architecture macroporeuse est constituée d'un réseau hexagonal principal de pores cylindriques (2) parallèles entre eux. Ces macropores ont un diamètre de 0.5 mm et ont leurs axes de symétrie distants de 1.5 mm. L'architecture macroporeuse de type hexagonale occupe 9 à 10% du volume apparent total hors canal central. La formulation du ciment brushite utilisé a une microporosité intrinsèque qui occupe environ 31% du volume total hors macroporosité. Le substitut a une porosité totale d'environ 37 à 38% en volume.

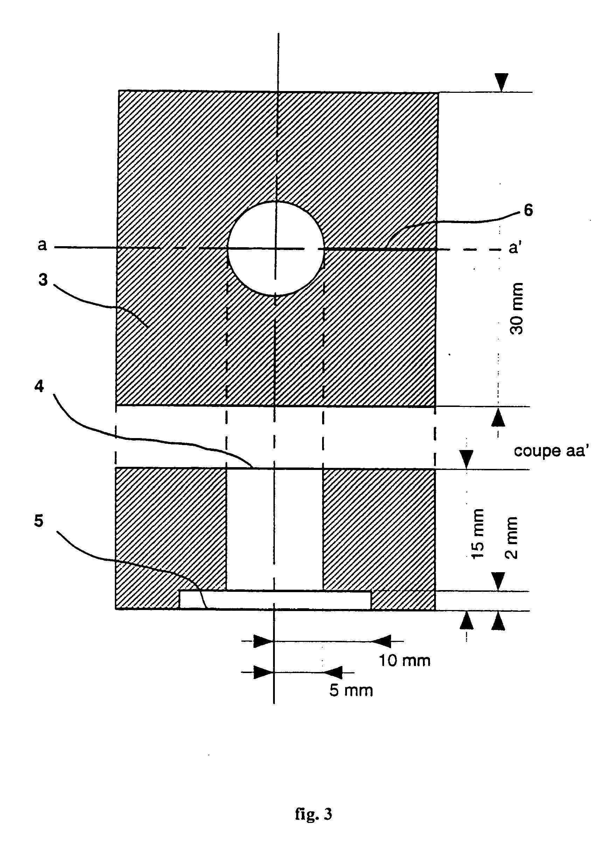

### Description des éléments du dispositif (fig. 3, 4 et 5)

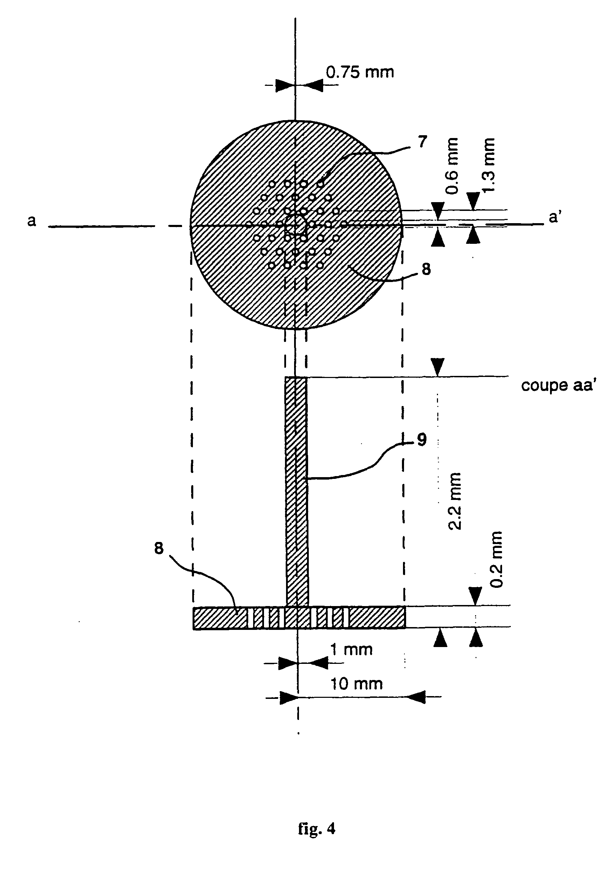

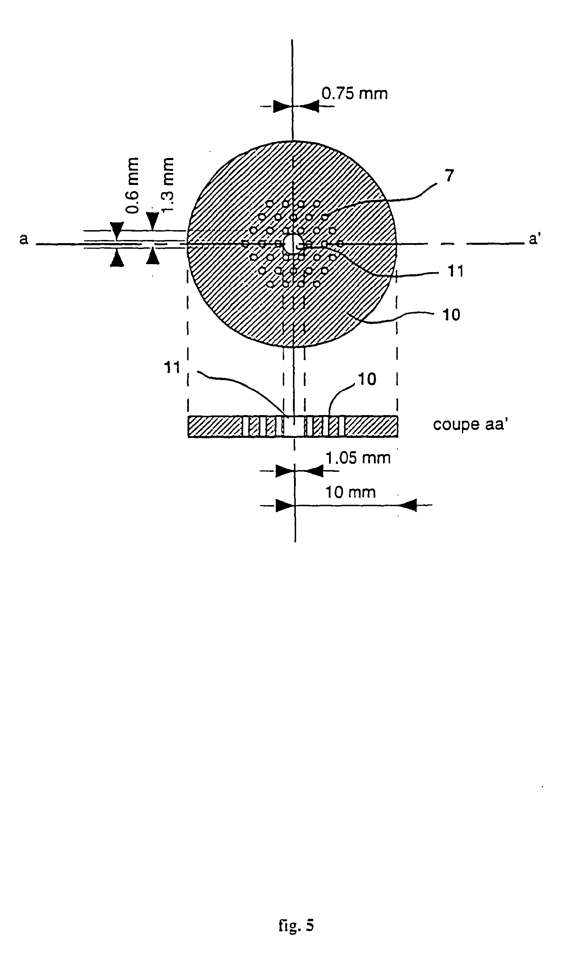

Le moule (3) est réalisé d'une seule pièce en caoutchouc de silicone par simple coulage dans un contre-moule en acier. Il comporte deux ouvertures de diamètres différents (4, 5) et une fente latérale (6) (fig. 3). Le dispositif utilisé pour réaliser la macroporosité unidirectionnelle est constitué de deux pièces d'aluminium (fig. 4 et 5) toutes deux préalablement percées de 36 trous (7) de 0.6 mm de diamètre et dont les centre de symétrie sont distants de 1.5 mm. C'est par ces trous que passeront des brins de fil en polymère de 0.5 mm de diamètre et de 40 mm de long. Une tige (9) de 2 mm de diamètre est fixée dans la plaque inférieure (8) alors que la plaque supérieure(10) comporte un orifice central (11) de 2.1 mm de diamètre (fig. 4 et 5).

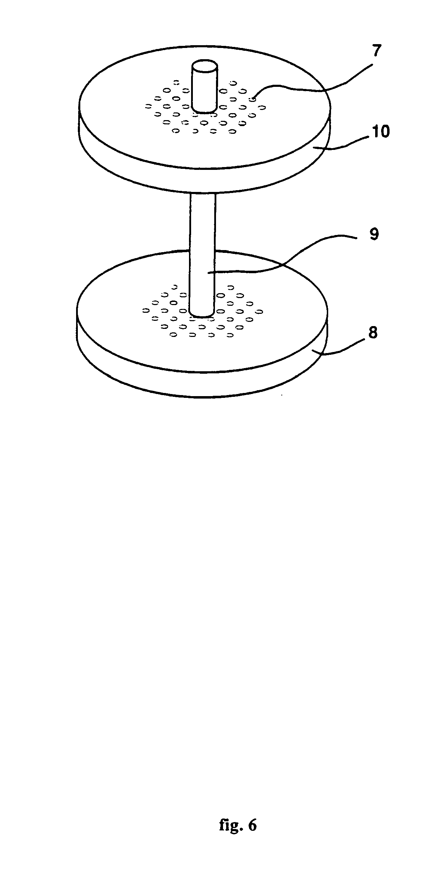

### Mise en oeuvre du dispositif (fig. 6, 7 et 8)

Le dispositif permettant de générer la macroporosité est conçu de manière à être réutilisable. La plaque (10) avec l'orifice central coulisse le long de la tige (9) fixée à la seconde plaque (8)(fig. 6). Les brins de fil de polymère de 40 mm de longueur sont enfilés au travers des 36 trous (7) de 0.6 mm de diamètre des deux plaques (8, 10). Ces brins sont ensuite fixés à la plaque (8) par encollage.

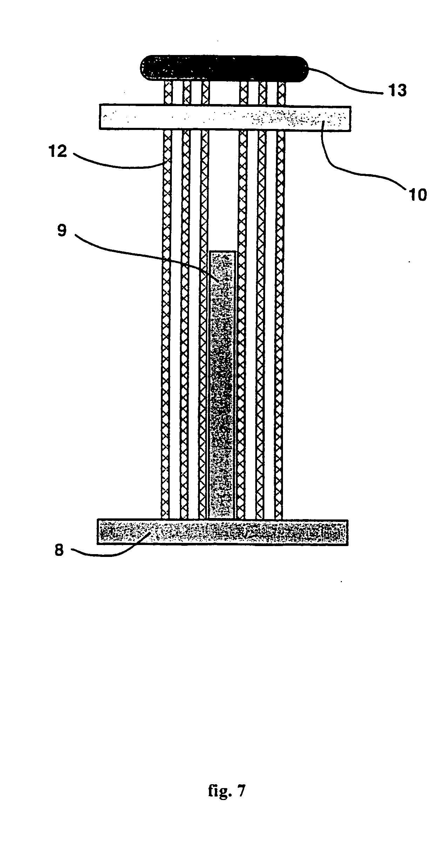

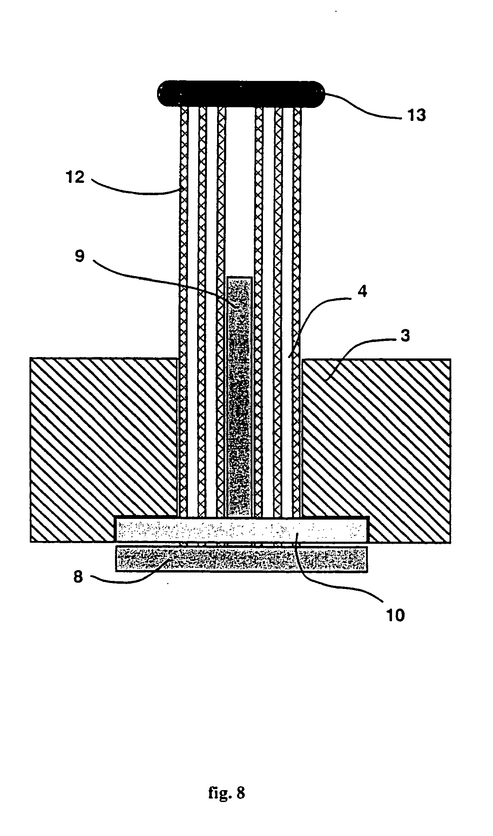

Ainsi la plaque (10) reste mobile et peut coulisser le long de la tige et du réseau de fils. Les brins de polymère (12) sont tendus entre les deux plaques afin de former un réseau de fils unidirectionnel. Leurs extrémités du côté de la plaque mobile (10) sont plongées dans un petit récipient de cire liquide. Une fois durcie, la cire (13) permet de maintenir les fils parallèles (fig. 7). La plaque mobile (10) est déplacée le long de la tige (9) et des fils (12) jusqu'à rejoindre la plaque (8) fixe. Ainsi, le réseau de fils (12) est maintenu par les deux plaques (8, 10) à une extrémité et par la cire durcie (13) à l'autre extrémité (fig. 8). Le réseau de fils est inséré dans le moule en caoutchouc de silicone (3) par la fente (6) ménagée sur le côté du moule (fig. 8). Le tout est ensuite centré par rapport à l'axe du moule : la plaque (10) est engagée dans l'ouverture inférieure (5) du moule en caoutchouc de silicone prévue à cette effet (fig. 8).

### Synthèse et moulage de la pâte de ciment brushite dans le moule

Le système associant deux poudres de calcium phosphate et de l'eau répond à la réaction suivante :

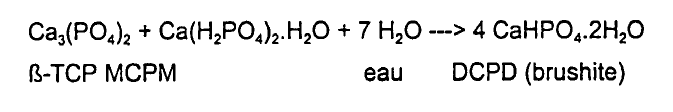

Cette réaction de précipitation de la brushite en présence d'eau donne lieu à la prise du ciment. En l'absence d'additifs, cette réaction est rapide et le ciment durcit en moins d'une minute, ce qui rend son utilisation peu pratique. L'emploi d'une solution diluée de H₂SO₄ (0.1 M) comme solution de gâchage combiné à un dosage précis d'additifs (pyrophosphate) permet un bon contrôle du temps de prise du ciment. Le temps de prise est ici ajusté entre 10 et 15 minutes pour faciliter la mise en oeuvre.

Le rapport solide/liquide du mélange est fixé à 2.5 g/ml afin d'obtenir une pâte plastique de viscosité adaptée à un bon moulage.
Le mélange consiste en :
- 1.2 g de poudre de phosphate tricalcique beta (β-TCP) dont le rapport Ca/P est compris entre 1.44 et 1.47. Le diamètre médian de cette poudre est 2.5 µm et sa surface spécifique de l'ordre de 1.5 m²/g.
- 0.8 g de poudre de phosphate monocalcique monohydraté (MCPM).
- 0.015 g de pyrophosphate de sodium (Na₂H₂P₂O₇).
- 1 ml d'une solution diluée d'H₂SO₄ (0.1 M).

L'acide sulfurique et les poudres de MCPM et Na₂H₂P₂O₇ sont soigneusement mélangées dans un mortier à l'aide d'une spatule. La poudre de β-TCP est ensuite ajoutée et le mélange est malaxé environ une minute jusqu'à l'obtention d'une pâte homogène. Il est à noter qu'à température ambiante (19-20°C), la pâte doit être utilisée dans les 10 minutes.

La pâte est disposée dans une seringue puis injectée dans le moule en caoutchouc de silicone par l'ouverture supérieure (4). Cette étape est faite en plusieurs fois afin de s'assurer du bon remplissage du moule. Entre deux ajouts successifs, le moule est vibré environ 30 secondes pour éliminer les bulles d'air qui pourraient subsister dans la pâte. Le moule est rempli à ras bord.

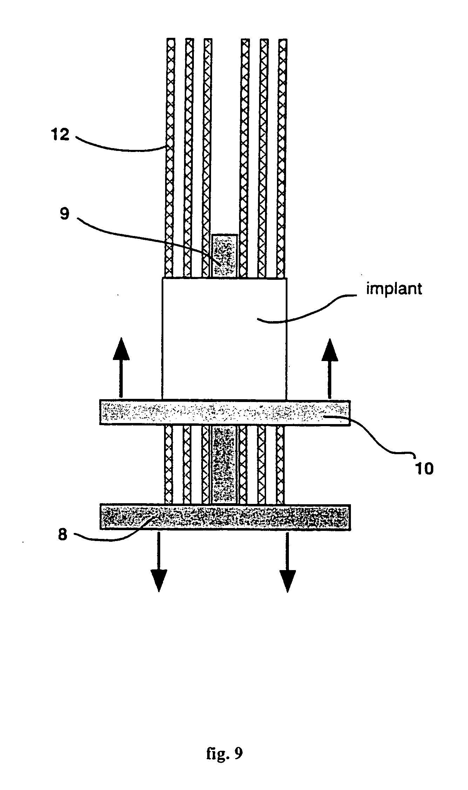

### Démoulage du substitut (fig. 9)

Une fois la consolidation du ciment terminée, le substitut est retiré du moule en caoutchouc de silicone par la fente (6). La pièce en cire (13) est ôtée, libérant ainsi les extrémités des brins de fil en polymère. Enfin, les brins de fil en polymère et la tige centrale sont soigneusement retirés du substitut par simple traction sur la base (8) en maintenant la plaque (10) fixe (fig. 9). La hauteur du substitut est rectifiée à 10 mm par polissage

### b) Substitut à architecture macroporeuse bidirectionnelle.

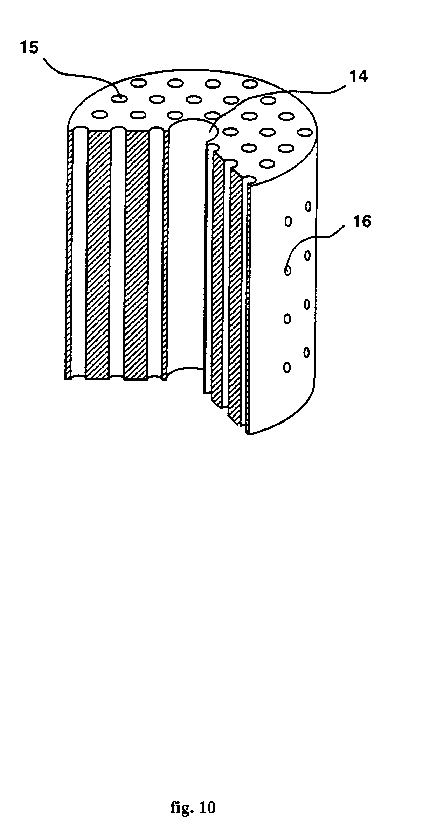

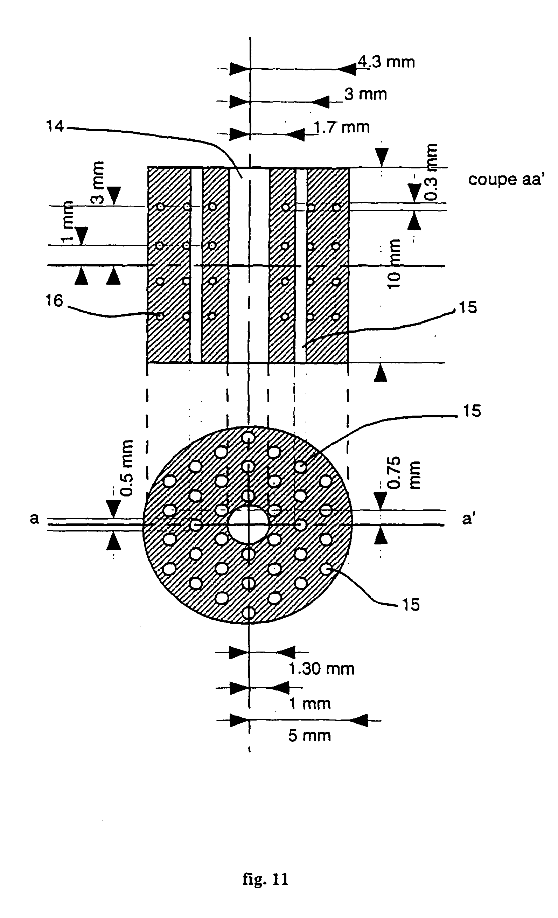

### Description du substitut (fig. 10 et 11)

Le substitut se présente sous forme d'un cylindre de 10 mm de diamètre et 10 mm de hauteur. Un canal central (14) de 2 mm de diamètre est pratiqué selon l'axe du substitut et simule la cavité médullaire de l'os à remplacer. L'architecture macroporeuse est constituée de deux réseaux de pores allongés interconnectés. Le réseau poreux principal (15) de type hexagonal est identique à celui des substituts à architecture macroporeuse unidirectionnelle. Il est constitué de pores cylindriques parallèles entre eux dont le diamètre est égal à 0.5 mm et dont les axes de symétrie sont distants de 1.5 mm. Les macropores cylindriques du réseau secondaire (16) ont un diamètre de 0.3 mm. Leurs axes de symétrie sont distants de 2 mm selon l'axe du substitut et de 1.3 mm selon les plans perpendiculaires à cet axe (coupe aa' de la figure 11). Le réseau poreux secondaire ne traverse pas le canal central (14) et se scinde de part et d'autre de celui-ci. L'architecture macroporeuse totale occupe une fraction volumique de 10.2%. La formulation du ciment brushite utilisé a une microporosité intrinsèque qui occupe environ 31% du volume total. Le substitut a une porosité totale d'environ 38% en volume.

### Description des éléments du dispositif (fig. 4, 5, 12, 13 et 14)

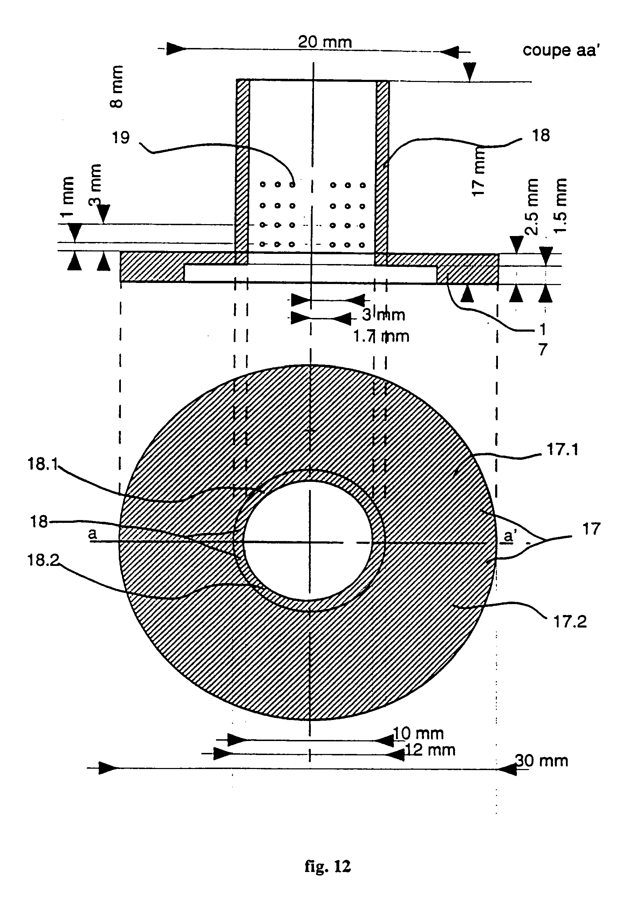

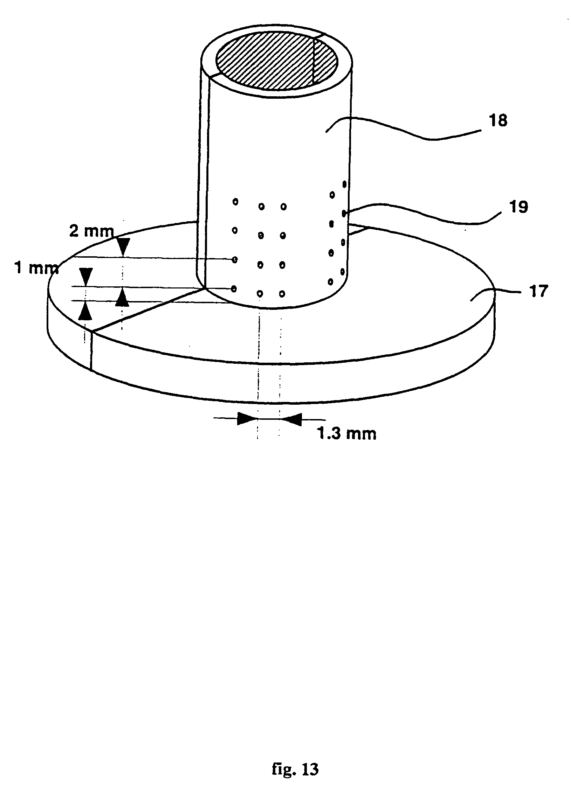

Le moule (fig. 12) en acier inoxydable est composé de quatre pièces : deux demi-disques (17.1 et 17.2) qui mis côte à côte forment un socle (17) de 30 mm de diamètre et de 2.5 mm d'épaisseur dans lequel est pratiqué un trou central de 10 mm de diamètre ; deux demi-cylindres (18.1 et 18.2) creux fixés chacun sur un demi-socle. Chaque assemblage (demi-disque plus demi-socle) forme un demi-moule en acier inoxydable. Le moule ainsi assemblé comporte donc un socle (17) muni d'un cylindre creux (18) de hauteur égale à 14.5 mm, de diamètre intérieur égale à 10 mm et dont l'épaisseur de la paroi est de 1 mm. Le cylindres creux (18) composé des deux demi-cylindres (18.1 et 18.2) est percé de part en part de 24 trous sphériques (19) de 0.35 mm de diamètre (fig. 12 et 13). Ces trous ont leurs axes de symétrie distants de 2 mm selon l'axe du cylindre et de 1.3 mm selon les plans perpendiculaires à cet axe. Le dispositif utilisé pour réaliser la macroporosité du réseau principal est identique à celui mis en oeuvre dans le cas des substituts à architecture macroporeuse unidirectionnelle (cf. ci-dessous et fig. 4 et 5). Il est constitué de deux pièces d'aluminium percées de 36 trous (7) de 0.6 mm de diamètre, dont les centres de symétrie sont distants de 1.5 mm et par lesquels passeront des brins de fil de polymère de 0.5 mm de diamètre et de 40 mm de long. Une tige (9) de 2 mm de diamètre est fixée dans la plaque inférieure (8) alors que la plaque supérieure (10) comporte un orifice central (11) de 2.1 mm de diamètre.

### Mise en oeuvre du dispositif (fig. 6, 7 et 14)

La première étape de mise en oeuvre correspond à celle utilisée pour les substituts à architecture macroporeuse unidirectionnelle (cf. ci-dessus et fig. 6, 7). Le réseau de fils unidirectionnel permettant de générer le réseau macroporeux principal est inséré entre les deux demi-moules en acier inoxydable. Le tout est ensuite centré par rapport à l'axe du moule : la plaque (10) est engagée dans l'ouverture inférieure du moule en acier inoxydable prévue à cette effet.

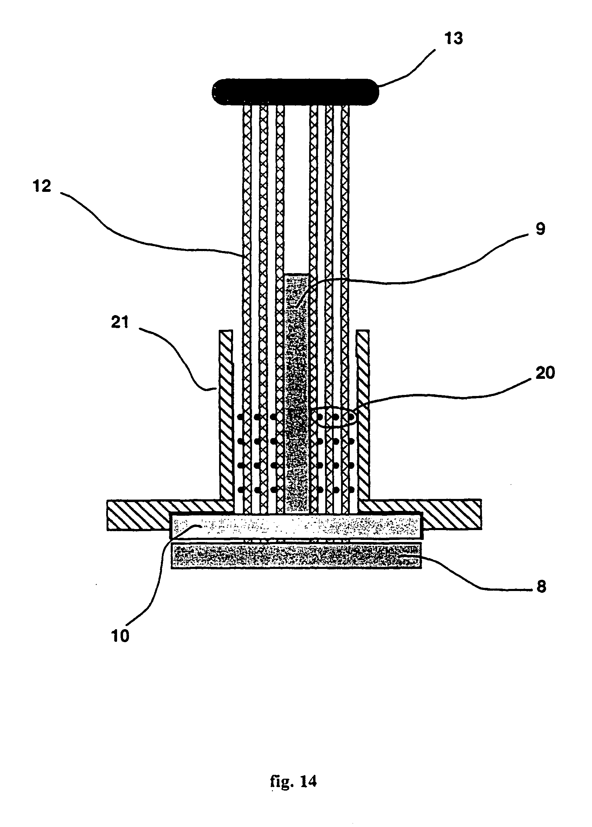

L'étape supplémentaire consiste donc à faire passer des brins de fil en acier inoxydable (20) de 0.3 mm de diamètre et de 30 mm de longueur au travers des 24 canaux (19) du moule en acier inoxydable (21) (fig. 14). Le tout forme un second réseau de fils en acier (20) interconnecté et perpendiculaire au réseau principal de fils en polymère (12). Chaque fil en acier vient au contact des fils en polymère de chaque rangée du premier réseau principal.

### Synthèse et moulage de la pâte de ciment brushite dans le moule

Ces deux étapes sont similaires à celles utilisées pour les substituts à architecture macroporeuses unidirectionnelle déjà décrites.

### Démoulage du substitut

Une fois la consolidation du ciment terminée, les brins de fil en acier inoxydable (20) sont retirés un à un de l'ensemble formé du moule et du substitut. Les deux demi-moules en acier inoxydable sont ôtés libérant le substitut. La pièce en cire (13) est enlevée, libérant ainsi les extrémités des brins de fil en polymère. Enfin, les brins de fil en polymère et la tige centrale sont soigneusement retirés du substitut par simple traction sur la base (8) en maintenant la plaque (10) fixe. La hauteur du substitut est rectifiée à 10 mm par polissage.

### EXEMPLE 2 : SUBSTITUT EN CIMENT PHOSPHOCALCIQUE DE FORME ANATOMIQUE ET D'ARCHITECTURE MACROPOREUSE REALISEES A PARTIR DU PROCEDE DE PROTYPAGE RAPIDE A JET DE CIRE (APPAREIL TYPE MODELMAKERII, SANDERS PROTOTYPE. INC).

Selon le cas, la forme de la pièce en cire réalisée par prototypage rapide est soit le positif soit le négatif de la forme anatomique du substitut. La phase porogène est également produite par prototypage rapide correspond toujours au négatif de l'architecture macroporeuse désirée du substitut. La conception de la géométrie du moule et de la phase porogène peut se faire par imagerie médicale numérisée ou à partir de logiciel CAD. L'architecture macroporeuse peut être complexe, par exemple consister en un réseau tridimensionnel de cylindres interconnectés. Par ailleurs, il est possible de modéliser l'architecture macroporeuse interconnectée afin d'adapter les propriétés mécaniques du substitut tout en favorisant sa recolonisation osseuse. Il est également possible d'introduire un canal central qui simule la cavité médullaire de l'os à remplacer dans le cas d'os longs.

### a) Substitut en ciment brushite.

Une pièce en cire est réalisée par prototypage rapide à jet de cire. Sa géométrie correspond au négatif de la forme anatomique et de l'architecture macroporeuse du substitut. Ainsi, la pièce en cire joue deux rôles : d'une part, elle définit la géométrie externe du substitut ; d'autre part, elle sert de phase porogène c'est-à-dire qu'une fois éliminée, elle laisse une empreinte dans le ciment correspondant à l'architecture macroporeuse désirée. Une ouverture supérieure est prévue lors de la conception de la pièce afin de permettre son remplissage par injection de la pâte en ciment brushite. Les étapes de synthèse de la pâte de ciment brushitique et du remplissage de la pièce en cire sont similaires à celles de l'exemple 1 déjà décrit. Une fois la consolidation chimique du ciment terminée, la cire est éliminée à basse température par dissolution à l'aide d'un solvant. Le substitut obtenu en ciment brushite a une forme anatomique et une architecture macroporeuse contrôlées à volonté.

### b) Substitut en ciment hydroxyapatite.

Le substitut est mis en forme par coulage d'une pâte liquide phosphocalcique dans un moule en plâtre. Deux demi-moules en plâtre sont réalisés en utilisant le procédé du moule à cire perdue. Pour se faire, deux pièces en cire correspondant aux positifs de deux demi-substituts, sont produites par prototypage rapide à jet de cire. Ces deux pièces sont ensuite plongées séparément dans du plâtre liquide : après consolidation du plâtre, les pièces en cire sont éliminées à l'aide d'un solvant ce qui permet d'obtenir les deux demi-moules en plâtre. Une troisième pièce en cire est produite par prototypage à jet de cire. Sa géométrie correspond au négatif de l'architecture macroporeuse du substitut et a été conçue de manière à s'insérer parfaitement dans le moule formé des deux demi-moules en plâtre. Le dispositif obtenu (deux demi-moules en plâtre et phase porogène) sert à la réalisation du substitut à partir du coulage d'une pâte liquide phosphocalcique. Cette pâte liquide est composée d'un mélange stoechiométrique de poudres de phosphate dicalcique anhydre (DCP) et de calcite (CC) en présence d'une solution dispersante d'acide polyacrylique à 2%pds et à raison de 0.5 ml par gramme de mélange des poudres (DCP/CC). Une alimentation en continue de la pâte liquide dans le dispositif permet de contrôler la hauteur de la pièce au fur et à mesure du retrait dû au ressuyage par le plâtre. Une fois le corps cru consolidé, le moule en plâtre est retiré et la phase porogène en cire est éliminée à basse température à l'aide d'un solvant. Le corps cru est ensuite transformé en autoclave. La réaction de consolidation se fait à 121°C pendant 1 heure et conduit à la précipitation d'hydroxyapatite accompagnée d'un dégagement de gaz carbonique et d'eau :

3CaHPO₄ + 2CaCO₃ => Ca₅(PO₄)₃OH + 2CO₂ + H₂O

DCP + CC => HAp

Le substitut en ciment hydroxyapatite obtenu a une architecture macroporeuse et une forme anatomique contrôlées à volonté.

## Revendications

1. Substitut osseux, réalisé en une pièce à partir de matériaux phosphocalciques cimentaires consolidés chimiquement à basse température et en présence d'eau, ledit substitut osseux comprenant un canal central (14), un réseau principal constitué d'une pluralité de canaux parallèles (15) disposés parallèlement et autour du canal central (14), **caractérisé par le fait qu'**il comprend en outre au moins un réseau secondaire constitué de canaux (16), ledit réseau secondaire étant interconnecté audit réseau principal.

2. Substitut osseux selon la revendication 1, **caractérisé par le fait que** la section des canaux (15) du réseau principal est comprise entre 8.10⁻³ et 0.8 mm², de préférence entre 0.1 et 0.3 mm².

3. Substitut osseux selon la revendication 1 ou 2, **caractérisé par le fait que** la section des canaux (16) du réseau secondaire est comprise entre 3 . 10⁻³ et 8 . 10⁻³ mm².

4. Substitut osseux selon l'une quelconque des revendications précédentes, **caractérisé par le fait que** la fraction volumique des canaux principaux et secondaires (15,16) est comprise entre 10 % et 70 % et de préférence entre 30 % et 60 %.

5. Substitut osseux selon l'une quelconque des revendications précédentes, **caractérisé par le fait que** la porosité totale du substitut est comprise entre 28 % et 88 %.

6. Substitut osseux selon l'une quelconque des revendications précédentes réalisé à partir d'une pâte liquide ou plastique qui comprend un ou plusieurs phosphates de calcium et au moins un autre sel de calcium peu soluble tels que sulfate, pyrophosphate ou carbonate.

7. Substitut osseux selon la revendication précédente réalisé à partir d'un mélange tel que phosphate tricalcique-β (β-TCP) et phosphate monocalcique monohydraté (MCPM), phosphate tétracalcique monoxyde (TTCP) et phosphate dicalcique dihydraté (DCPD), phosphate tricalcique-α (α-TCP) et phosphate monocalcique monohydraté (MCPM) ou phosphate dicalcique anhydre (DCP) et carbonate de calcium (calcite, aragonite ou vatérite).

8. Substitut osseux selon l'une quelconque des revendications précédentes réalisé en un matériau obtenu par étuvage en atmosphère saturée en humidité de mélange de monétite (phosphate dicalcique anhydre) et de carbonate de calcium (calcite, aragonite ou vatérite).

9. Procédé d'obtention d'un substitut osseux selon l'une quelconque des revendications précédentes, **caractérisé en ce que** la réaction de consolidation du ciment se fait chimiquement ex-vivo, soit à température et milieu ambiant, soit en milieu saturé en eau et à température plus élevée.

10. Procédé d'obtention d'un substitut osseux selon la revendication 9, **caractérisé en ce que** la géométrie anatomique et l'architecture macroporeuse sont réalisées durant la mise en forme par moulage de la pâte phosphocalcique et par consolidation chimique ex-vivo.

11. Procédé d'obtention d'un substitut osseux selon l'une quelconque des revendications 9 ou 10, **caractérisé par le fait que** les canaux principaux et secondaires (15,16) sont réalisés en utilisant des fils en polymère (12) tels que nylon, PVB ou polyéthylène.

12. Procédé d'obtention d'un substitut osseux selon l'une quelconque des revendications précédentes 9 à 11, **caractérisé par** l'utilisation d'un procédé de prototypage rapide, tel que la stéréolithographie ou le prototypage rapide par jet de cire, pour la réalisation du moule et de la phase porogène.

## Claims

1. Bone substitute made in one piece from cementitious calcium phosphate materials chemically consolidated at low temperature in the presence of water, said bone substitute comprising a central channel (14), a main network composed of a plurality of parallel channels (15) arranged parallel to and around the central channel (14), **characterised by** the fact that it additionally comprises at least one secondary network composed of channels (16), said secondary network being interconnected to said main network.

2. Bone substitute according to claim 1, **characterised by** the fact that the channel (15) section of the main channel network is comprised between 8.10⁻³ et 0.8 mm², preferably between 0.1 et 0.3 mm².

3. Bone substitute according to either claim 1 or 2, **characterised by** the fact that the channel (16) sections of the secondary channel network is comprised between 3 . 10⁻³ et 8 . 10⁻³ mm².

4. Bone substitute according to any of the previous claims, **characterised by** the fact that the volume fraction of the main and secondary channels (15,16) is comprised between 10 % and 70 %, preferably between 30 % and 60 %.

5. Bone substitute according to any of the previous claims, **characterised by** the fact that the total pore volume fraction is comprised between 28 % and 88 %.

6. Bone substitute according to any of the previous claims, made from a liquid or plastic paste comprising at least one calcium phosphate and at least one further sparingly soluble calcium salt such as sulfate, pyrophosphate or carbonate.

7. Bone substitute according to the previous claim, obtained from a mixture such as : β-tricalcium phosphate (β-TCP) and monocalcium phosphate monohydrate (MCPM); tetracalcium phosphate monoxide (TTCP) and dicalcium phosphate dihydrate (DCPD); α-tricalcium phosphate (α-TCP) and monocalcium phosphate monohydrate (MCPM); dicalcium phosphate anhydrous (DCPA) and calcium carbonate (calcite, aragonite or vaterite).

8. Bone substitute according to any of the previous claims, made of a material obtained by heating a mixture of monetite (dicalcium phosphate anhydrous) and calcium carbonate (calcite, aragonite or vaterite) in a water-saturated atmosphere.

9. Process for manufacturing a bone substitute according to any of the previous claims, **characterised by** the fact that the cementitious mixture is consolidated through a chemical process taking place ex-vivo, either in ambient atmosphere at ambient temperature, or at higher temperatures in a water-saturated atmosphere.

10. Process for manufacturing a bone substitute according to claim 9, **characterised by** the fact that the anatomic shape and the macroporous architecture of said bone substitute are obtained during the moulding process of a calcium phosphate cementitious paste and the chemical consolidation ex-vivo of said cementitious paste.

11. Process for manufacturing a bone substitute according to either claim 9 or 10, **characterised by** the fact that the main and secondary channels (15,16) are made using threads (12) made of polymers such as nylon, PVB or polyethylene.

12. Process for manufacturing a bone substitute according to any of the previous claims 9 to 11, **characterised by** the use of a rapid prototyping technique, such as stereolithography or fused-jet rapid prototyping, for making the mould and the porogenic architecture of said bone substitute.

## Patentansprüche

1. Knochenersatz, hergestellt aus einem Stück ausgehend von Calciumphosphat-Zementmaterialien, die bei niedriger Temperatur chemisch verfestigt werden, und in Gegenwart von Wasser, wobei der Knochenersatz einen zentralen Kanal (14) und ein hauptsächliches Netzwerk aus einer Mehrzahl paralleler Kanäle (15) umfasst, die parallel und um den zentralen Kanal (14) herum angeordnet sind, **dadurch gekennzeichnet, dass** er ferner mindestens ein sekundäres, aus Kanälen (16) bestehendes Netzwerk umfasst, wobei das sekundäre Netzwerk mit dem hauptsächlichen Netzwerk vernetzt ist.

2. Knochenersatz nach Anspruch 1, **dadurch gekennzeichnet, dass** der Querschnitt der Kanäle (15) des hauptsächlichen Netzwerks zwischen 8 × 10⁻³ und 0,8 mm², vorzugsweise zwischen 0,1 und 0,3 mm², beträgt.

3. Knochenersatz nach Anspruch 1 oder 2, **dadurch gekennzeichnet, dass** der Querschnitt der Kanäle (16) des sekundären Netzwerks zwischen 3 × 10⁻³ und 8 × 10⁻³ mm² liegt.

4. Knochenersatz nach einem der vorhergehenden Ansprüche, **dadurch gekennzeichnet, dass** die Volumenfraktion der hauptsächlichen und sekundären Kanäle (15, 16) zwischen 10 und 70%, vorzugsweise zwischen 30 und 60%, beträgt.

5. Knochenersatz nach einem der vorhergehenden Ansprüche, **dadurch gekennzeichnet, dass** die Gesamtporosität des Ersatzes zwischen 28% und 88% beträgt.

6. Knochenersatz nach einem der vorhergehenden Ansprüche, der aus einer flüssigen oder plastischen Masse hergestellt wird, die ein oder mehrere Calciumphosphate und mindestens ein anderes wenig lösliches Calciumsalz, wie Sulfat, Pyrophosphat oder Carbonat, umfasst.

7. Knochenersatz nach dem vorhergehenden Anspruch, der aus einem Gemisch, wie β-Tricalciumphosphat (β-TCP) und Monocalciumphosphatmonohydrat (MCPM), Tetracalciumphosphatmonoxid (TTCP) und Dicalciumphosphatdihydrat (DCPD), α-Tricalciumphosphat (α-TCP) und Monocalciumphosphatmonohydrat (MCPM) oder wasserfreiem Dicalciumphosphat (DCP) und Calciumcarbonat (Calcit, Aragonit oder Vaterit), hergestellt wird.

8. Knochenersatz nach einem der vorhergehenden Ansprüche, der aus einem Material hergestellt wird, das durch Tempern eines Gemischs aus Monetit (wasserfreiem Dicalciumphosphat) und Calciumcarbonat (Calcit, Aragonit oder Vaterit) in einer feuchtigkeitsgesättigten Atmosphäre erhalten wird.

9. Verfahren zur Gewinnung eines Knochenersatzes nach einem der vorhergehenden Ansprüche, **dadurch gekennzeichnet, dass** die Reaktion der Verfestigung des Zements chemisch ex vivo entweder bei Umgebungstemperatur und -bedingungen oder in einer wassergesättigten Umgebung und bei erhöhter Temperatur erfolgt.

10. Verfahren zur Gewinnung eines Knochenersatzes nach Anspruch 9, **dadurch gekennzeichnet, dass** die anatomische Geometrie und makroporöse Architektur bei der Formung durch Formen der Calciumphosphat-Masse und durch chemische ex-vivo-Verfestigung hergestellt werden.

11. Verfahren zur Gewinnung eines Knochenersatzes nach einem der Ansprüche 9 oder 10, **dadurch gekennzeichnet, dass** die hauptsächlichen und sekundären Kanäle (15, 16) unter Verwendung von Fäden aus Polymer (12), wie Nylon, PVB oder Polyethylen, hergestellt werden.

12. Verfahren zur Gewinnung eines Knochenersatzes nach einem der vorhergehenden Ansprüche 9 bis 11, **gekennzeichnet durch** Verwendung eines Schnellprototypisierungsverfahrens, wie Stereolithographie oder Wachsstrahl-Schnellprototypisierung, zur Herstellung der Form und der porogenen Phase.
